(19) Europäisches Patentamt
European Patent Office
Office européen des brevets

(11) **EP 4 454 714 A1**

(12) **EUROPEAN PATENT APPLICATION**

(43) Date of publication:
**30.10.2024 Bulletin 2024/44**

(21) Application number: **23170654.0**

(22) Date of filing: **28.04.2023**

(51) International Patent Classification (IPC):
**A61Q 5/06** (2006.01)    **A61K 8/34** (2006.01)
**A61K 8/73** (2006.01)

(52) Cooperative Patent Classification (CPC):
**A61Q 5/06; A61K 8/345; A61K 8/732;**
A61K 2800/5422; A61K 2800/87; A61K 2800/882

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Kao Corporation**
**Chuo-ku**
**Tokyo 103-8210 (JP)**

(72) Inventors:
• **PYROZOK, Erik**
  **Tokyo, 103-8210 (JP)**
• **EZURE, Mikako**
  **Tokyo, 103-8210 (JP)**
• **IMAI, Megumi**
  **Tokyo, 103-8210 (JP)**

(74) Representative: **Hoffmann Eitle**
**Patent- und Rechtsanwälte PartmbB**
**Arabellastraße 30**
**81925 München (DE)**

Remarks:
Amended claims in accordance with Rule 137(2)
EPC.

(54) **METHOD FOR CARING AND/OR STYLING OF KERATIN FIBERS**

(57) The present invention is directed to a method for caring and/or styling keratin fibers, preferably human keratin fibers, more preferably human hair, comprising: i) applying to keratin fibers an aqueous composition A comprising a) one or more organic solvent(s), b) one or more non-ionic hair styling polymer(s), ii) leaving the composition onto the keratin fibers and drying the keratin fibers, and iii) mechanically treating the keratin fibers, preferably mechanically curling the keratin fibers, more preferably mechanically curling the keratin fibers by scrunching, squeezing, or twisting the keratin fibers, wherein the total concentration of anionic polymers in composition A is 0.2% by weight or less, preferably 0.1% by weight or less, more preferably 0.05% by weight or less, calculated to the total weight of the composition A, more preferably composition A is free of anionic polymers.

**Description**

Field of the Invention

The present invention is directed to a method for caring and/or styling of keratin fibers. A kit-of-parts comprising a cosmetic composition and a dispenser is disclosed.

Background of the Invention

The use of hair styling products is widely spread among curly-hair customers. However, a common problem is that these products lead to undesired side effects such as sticky feeling of hair, blunt appearance, and unnatural weight-down. Moreover, for customers desiring an improvement of their natural hair curls, the curling results tends to look unnatural or artificial.

Mintel 1843414 and 8756781 disclose hair styling products comprising an organic solvent and non-ionic styling polymer. However, the products comprise anionic polymers impairing the curl definition properties in combination with mechanical treatment of the hair.

Thus, there is a real customer need for delivering caring and/or styling methods that improve the appearance of their curls while also ensuring a natural look and keeping the natural movement of curls, while not weighing down the hair.

Summary of the Invention

Therefore, the first object of the present invention is a method for caring and/or styling keratin fibers, preferably human keratin fibers, more preferably human hair, comprising:

> i) applying to keratin fibers an aqueous composition A comprising:
>
>> a) one or more organic solvent(s),
>> b) one or more non-ionic hair styling polymer(s),
>
> ii) leaving the composition onto the keratin fibers and drying the keratin fibers, and
> iii) mechanically treating the keratin fibers, preferably mechanically curling the keratin fibers, more preferably mechanically curling the keratin fibers by scrunching, squeezing, or twisting the keratin fibers,
> wherein the total concentration of anionic polymers in composition A is 0.2% by weight or less, preferably 0.1% by weight or less, more preferably 0.05% by weight or less, calculated to the total weight of the composition A, more preferably composition A is free of anionic polymers.

The second object of the present invention is a kit-of-parts for caring and/or styling keratin fibers, preferably human keratin fibers, more preferably human hair, comprising:

- a composition B comprising one or more organic solvent(s) as compound(s) according to group a) as defined above,
- a composition C comprising one or more non-ionic hair styling polymer(s) as compound(s) according to group b) as defined above,

with the provision that at least one of the compositions B and/or C are aqueous compositions, and
wherein the total concentration of anionic polymers in compositions B and C is 0.2% by weight or less, preferably 0.1% by weight or less, more preferably 0.05% by weight or less, calculated to the total weight of each of the compositions B and C, more preferably compositions B and C are free of anionic polymers.

The third object of the present invention is a dispenser comprising the composition A as defined above or the compositions B and C as defined above.

Detailed Description of the Invention

The inventors of the present invention have unexpectedly found out that the objects of the independent claims provide for an improved curl effect that looked natural, did not weigh down the hair, and had a natural appearance.

The present invention is directed to a method for caring and/or styling keratin fibers, preferably human keratin fibers, more preferably human hair, comprising:

i) applying to keratin fibers an aqueous composition A comprising:

    a) one or more organic solvent(s),
    b) one or more non-ionic hair styling polymer(s),

ii) leaving the composition onto the keratin fibers and drying the keratin fibers, and
iii) mechanically treating the keratin fibers, preferably mechanically curling the keratin fibers, more preferably mechanically curling the keratin fibers by scrunching, squeezing, or twisting the keratin fibers,
wherein the total concentration of anionic polymers in composition A is 0.2% by weight or less, preferably 0.1% by weight or less, more preferably 0.05% by weight or less, calculated to the total weight of the composition A, more preferably composition A is free of anionic polymers.

Composition A

Composition A is an aqueous composition. The term 'aqueous' within the meaning of the present invention denotes a composition A comprising water at 50% by weight, preferably at 75% by weight, more preferably at 80% by weight, calculated to the total weight of composition A.
It is preferred from the viewpoint of cosmetic compatibility that the pH of composition A is 3 or more, preferably 4 or more, still more preferably 4.5 or more.
It is preferred from the viewpoint of cosmetic compatibility that the pH of composition A is 9 or less, preferably 7 or less, still more preferably 6.5 or less.
For attaining the above-mentioned effect, it is preferred that the pH of composition A is in the range of 3 to 9, preferably the pH is in the range of 4 to 7, more preferably the pH is in the range of 4.5 to 6.5.
The pH of the composition may be adjusted with all cosmetically acceptable acids or bases. Suitable acids are, for example citric acid, malic acid, fumaric acid, succinic acid, lactic acid, and hydrochloric acid. Suitable bases are, for example, monoethanolamine, earth alkaline or alkali salts of carbonate or bicarbonate, and sodium hydroxide.
Composition A further comprises one or more organic solvent(s) as compound(s) according to group a). It is preferred from the viewpoint of curl definition that the one or more organic solvent(s) are selected from monohydric, dihydric, and/or trihydric alcohol(s), and/or their mixtures.
Suitable monohydric alcohols are ethanol, propanol, isopropanol, butanol, and isobutanol, and ethoxydiglycol.
Suitable dihydric and/or trihydric alcohols are, for example, 1,2-propanediol, 1,3-propanediol, glycerol.
It is preferred from the viewpoint of curl definition that one or more organic solvent(s) as compound(s) according to group a) is/are selected from monohydric, dihydric, and/or trihydric alcohol(s), and/or their mixtures, more preferably one or more organic solvent(s) as compound(s) according to group a) is/are selected from ethanol, propanol, isopropanol, butanol, isobutanol, ethoxydiglycol, 1,2-propanediol, 1,3-propanediol, and glycerol, and/or their mixtures, still more preferably one or more organic solvent(s) as compound(s) according to group a) is/are selected from 1,3-propanediol, ethoxydiglycol, and glycerol, and/or their mixtures, still more preferably the one or more organic solvent(s) as compound(s) according to group a) is 1,3-propanediol, glycerol, and/or their mixtures.
It is preferred from the viewpoint of curl definition that the total concentration of one or more compound(s) according to group a) in composition A, preferably the total concentration of 1,3-propanediol and/or glycerol, and/or their mixtures, in the composition A, is 0.1% by weight or more, preferably 0.25% by weight or more, still more preferably 0.5% by weight or more, calculated to the total weight of composition A.
It is preferred from the viewpoint of curl definition that the total concentration of one or more compound(s) according to group a) in composition A, preferably the total concentration of 1,3-propanediol and/or glycerol, and/or their mixtures, in the composition A, is 10% by weight or less, preferably 7.5% by weight or less, still more preferably 5% by weight or less, calculated to the total weight of composition A.
For attaining the above-mentioned effects, it is preferred that the total concentration of one or more compound(s) according to group a) in composition A, preferably the total concentration of 1,3-propanediol and/or glycerol, and/or their mixtures, in the composition A, is in the range of 0.1% to 10% by weight, preferably in the range of 0.25% to 7.5% by weight, more preferably in the range of 0.5% to 5% by weight, calculated to the total weight of the composition A.
Composition A comprises one or more non-ionic polymer(s) of composition A as compound(s) according to group b).
Suitable non-ionic styling polymers are, for example, vinylpyrrolidone polymers of either homopolymers or copolymers with, particularly, vinylacetate, commonly known as VP/VA copolymers. Those homopolymers are known with the trade name "Luviskol" (registered trade mark) as Luviskol (registered trade mark) K 30, K 60 or K 90, whereas the VP/VA copolymers are known as Luviskol (registered trade mark) VA 55, VA 64, or Plus from BASF AG.
Carbohydrate-based non-ionic hair styling polymers are particularly preferred for composition A from the viewpoint of curl definition. Carbohydrate-based polymers comprises one or more sugar units such as cellulose, and partially synthetic derivatise such as hydroxyethylcellulose or hydroxypropylcellulose, and starch-based polymers.

Hydrolyzed starch polymers are particularly preferred polymers for composition A from the viewpoint of curl definition and biodegradability. Starch polymers may be obtained from plants such as potato, wheat, rice, tapioca, or corn.

Thus, the most preferred one or more non-ionic polymer(s) as compound(s) according to group b) are selected from one or more hydrolyzed starch polymer(s), preferably hydrolyzed corn starch polymer(s).

It is preferred from the viewpoint of long-lastingness that the total concentration of one or more compound(s) according to group b) in composition A is 0.5% by weight or more, more preferably 0.75% by weight or more, still more preferably 1% by weight or more, calculated to the total weight of composition A.

It is preferred from the viewpoint of weigh-down effect that the total concentration of one or more compound(s) according to group b) in composition A is 15% by weight or less, more preferably 10% by weight or less, still more preferably 5% by weight or less, calculated to the total weight of composition A.

For attaining the above-mentioned effects, it is preferred that the total concentration of one or more compound(s) according to group b) in composition A is in the range of 0.5% to 15% by weight, preferably in the range of 0.75% to 10% by weight, more preferably in the range of 1% to 5% by weight, calculated to the total weight of composition A.

It is preferred from the viewpoint of dispensing that composition A comprises one or more foaming surfactant(s), preferably one or more non-ionic foaming surfactant(s) as detailed below.

It is preferred from the viewpoint of wetting that composition A comprises one or more surfactant(s), preferably one or more non-ionic, anionic, cationic, or amphoteric/zwitterionic surfactant(s), and/or their mixtures, more preferably one or more non-ionic surfactant(s).

Suitably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of $C_{10}$ to $C_{22}$ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of $C_{12}$ to $C_{22}$ or surfactants having a tertiary amine group and at least one alkyl chain having a carbon chain length in the range of $C_{12}$ to $C_{22}$ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of composition A, from the viewpoint of enhancing wettability of keratin fibers.

It is further preferred from the viewpoint of biodegradability that the total concentration of organopolysiloxanes in composition A is 0.5% by weight or less, preferably 0.1% by weight or less, more preferably composition A is free of organopolysiloxanes.

Organopolysiloxanes within the meaning of the present invention are organic compounds having siloxane repeating units.

Further method steps

It is preferred from the viewpoint of user convenience that the keratin fibers are shampooed prior to application of composition A onto the keratin fibers.

Independent of shampooing, it is preferred from the viewpoint of wettability that composition A is applied onto wet or damp keratin fibers.

Kit-of-parts

The present invention is also directed to a kit-of-parts for caring and/or styling keratin fibers, preferably human keratin fibers, more preferably human hair, comprising:

- a composition B comprising one or more organic solvent(s) as compound(s) according to group a) as defined above,
- a composition C comprising one or more non-ionic hair styling polymer(s) as compound(s) according to group b) as defined above,

with the provision that at least one of the compositions B and/or C are aqueous compositions, and

wherein the total concentration of anionic polymers in compositions B and C is 0.2% by weight or less, preferably 0.1% by weight or less, more preferably 0.05% by weight or less, calculated to the total weight of each of the compositions B and C, more preferably compositions B and C are free of anionic polymers.

The term 'aqueous' has the same definition as above for composition A.

It is preferred from the viewpoint of convenience that compositions B and C are kept separate until directly prior to application onto keratin fibers. Compositions B and C may either be mixed or be applied subsequently one after the other without a rinsing step.

Suitably, the total concentration of one or more organic solvents as compound(s) according to group a), preferably the total concentration of 1,3-propanediol in composition B, is in the range of 0.2% to 20% by weight, preferably in the range of 0.5% to 15% by weight, more preferably in the range of 1% to 10% by weight, calculated to the total weight of the composition B.

Suitably, the total concentration of one or more organic solvents as compound(s) according to group b) in composition C is in the range of 1% to 30% by weight, preferably in the range of 1.5% to 20% by weight, more preferably in the range of 2% to 10% by weight, calculated to the total weight of composition C.

The other compounds listed for composition A may also be present in compositions B and C in the same amounts as in composition A.

Dispenser

The present invention is also directed to a dispenser comprising the composition A as defined above or the compositions B and C as defined above.

Suitably, dispenser is an aerosol vaporizer or a non-aerosol vaporizer such as a pump spray dispenser, a pump foam dispenser or a squeeze foam dispenser, preferably the dispenser is a non-aerosol vaporizer such as a pump foam dispenser, more preferably the dispenser is a pump spray or pump foam dispenser.

From the viewpoint of environmental protection, the pump spray or pump foam dispenser are preferred.

For a dispenser comprising the compositions B and C, it is preferred that the compositions are dispensed from a dispenser having two chambers separated from each other. Mixing of compositions A and B can then be done prior to the dispensing or on the keratin fibers by dispensing compositions A and B separately, one after the other.

The following examples are to illustrate the present invention and not to limit it.

EXAMPLES

The following composition A1 was prepared by conventional mixing and dissolution techniques:

|  | % by weight |
|---|---|
| 1,3-propanediol | 1.0 |
| Hydrolyzed corn starch | 3.5 |
| NaOH/HCl | ad pH 5.5 |
| Water | ad 100.0 |

The following composition A2 was prepared by conventional mixing and dissolution techniques:

|  | % by weight |
|---|---|
| Glycerol | 1.0 |
| Hydrolyzed corn starch | 3.5 |
| NaOH/HCl | ad pH 5.5 |
| Water | ad 100.0 |

The following comparative composition 1 (Comparative 1) was prepared:

|  | % by weight |
|---|---|
| 1,3-propanediol | 1.0 |
| Hydrolyzed corn starch | 3.5 |

(continued)

|  | % by weight |
|---|---|
| Xanthan gum | 0.5 |
| NaOH/HCl | ad pH 5.5 |
| Water | ad 100.0 |

The following comparative composition 2 (Comparative 2) was prepared:

|  | % by weight |
|---|---|
| 1,3-propanediol | 1.0 |
| Hydrolyzed corn starch | 3.5 |
| Acacia gum | 0.5 |
| NaOH/HCl | ad pH 5.5 |
| Water | ad 100.0 |

EXAMPLES

Table 1

| Method steps | Inv. ex. 1 | Inv. ex. 2 | Inv. ex. 3 | Inv. ex. 4 | Inv. ex. 5 | Comp. ex. 1 | Comp. ex. 2 | Comp. ex. 3 |
|---|---|---|---|---|---|---|---|---|
|  | Comp. A1 | | | | Comp. A2 | Comp. A1 | Comparative 1 | Comparative 2 |
| Combing | + | - | - | - | - | - | - | - |
| Scrunching (10 x) | + | + | - | - | + | - | + | + |
| Squeezing (1 x) | - | - | + | - | - | - | - | - |
| Twisting around finger | - | - | - | + | - | - | - | - |
| Spiral probability [%] | 62 ± 5 | 62 ± 4 | 67 ± 9 | 59 ± 13 | 78 ± 11 | 40 ± 6 | 35 ± 8 | 43 ± 8 |
|  |  |  |  |  |  |  |  |  |

The mechanical treatment steps applied to the hair tress increased the spiral probability in comparison to the hair tress not mechanically treated (comparative example 1). The addition of high amounts of anionic polymers (comparative examples 2-3) reduced the spiral probability despite the mechanical treatment of the hair tress.

Methods

Preparation of hair tress

Caucasian, curly hair tress (20-30cm long, 2 g per bundle) ware shampooed with 0.2g of a commercial shampoo available under the brand name Kerasilk Reconstruct Shampoo. The hair tress were then rinsed off for 10s with lukewarm water. The water was allowed to drip for 10 s, then 0.5g one of the compositions from above were applied onto the wet hair tress.

Mechanical treatment of hair tress

After application of the treatment compositions, the hair tress were mechanically treated with a comb, by scrunching the hair tress (10 x), by squeezing the hair tress (1 x), and/or by twisting the hair tress around a finger from tip to root.

Measurement of spiral probability

The hair tress were allowed to air-dry and placed on a sheet of paper for evaluation. The numbers of o-curves (continuous spiral) corresponds to better bundling during drying, provides the hair with more bounce, and confers a more attractive visual appearance to customers. S-curves (discontinuous spiral), however, lead to frizz during drying resulting in non-cosmetic visual appearance of the hair. S-curves and o-curves were counted. The spiral probability was calculated as follows:

$$Spiral\ probability\ [\%] = \frac{n_{o-curves}}{n_{s-curves}} \cdot 100\%$$

Example 6

The following composition A can be prepared by conventional mixing and dissolution techniques:

|  | % by weight |
|---|---|
| 1,3-propanediol | 1.0 |
| Hydrolyzed corn starch | 3.5 |
| Olive oil | 0.5 |
| Tween 20 | 2.0 |
| NaOH/HCl | ad pH 5.5 |
| Water | ad 100.0 |

Example 7

The following composition B can be prepared by conventional mixing and dissolution techniques:

|  | % by weight |
|---|---|
| 1,3-propanediol | 1.0 |
| NaOH/HCl | ad pH 5.5 |
| Water | ad 100.0 |

The following composition C can be prepared by conventional mixing and dissolution techniques:

|  | % by weight |
|---|---|
| Hydrolyzed corn starch | 3.5 |
| Olive oil | 0.5 |
| Tween 20 | 2.0 |
| NaOH/HCl | ad pH 5.5 |
| Water | ad 100.0 |

Claims

1. A method for caring and/or styling keratin fibers, preferably human keratin fibers, more preferably human hair, comprising:

i) applying to keratin fibers an aqueous composition A comprising:

a) one or more organic solvent(s),
b) one or more non-ionic hair styling polymer(s),

ii) leaving the composition onto the keratin fibers and drying the keratin fibers, and
iii) mechanically treating the keratin fibers, preferably mechanically curling the keratin fibers, more preferably mechanically curling the keratin fibers by scrunching, squeezing, or twisting the keratin fibers,
wherein the total concentration of anionic polymers in composition A is 0.2% by weight or less, preferably 0.1% by weight or less, more preferably 0.05% by weight or less, calculated to the total weight of the composition A, more preferably composition A is free of anionic polymers.

2. The method according to claim 1 **characterized in that** the one or more organic solvent(s) as compound(s) according to group a) is/are selected from monohydric, dihydric, and/or trihydric alcohol(s), and/or their mixtures, preferably the one or more organic solvent(s) as compound(s) according to group a) is/are selected from ethanol, propanol, isopropanol, butanol, isobutanol, ethoxydiglycol, 1,2-propanediol, 1,3-propanediol, and glycerol, and/or their mixtures, more preferably the one or more organic solvent(s) as compound(s) according to group a) is/are selected from 1,3-propanediol, ethoxydiglycol, and glycerol, and/or their mixtures, still more preferably the one or more organic solvent(s) of composition A as compound(s) according to group a) is/are 1,3-propanediol, glycerol, and/or their mixtures.

3. The method according to any of the claims 1 and/or 2 **characterized in that** the total concentration of the one or more compound(s) according to group a) in composition A, preferably the total concentration of 1,3-propanediol in composition A, is in the range of 0.1% to 10% by weight, preferably in the range of 0.25% to 7.5% by weight, more preferably in the range of 0.5% to 5% by weight, calculated to the total weight of the composition A.

4. The method according to any of the preceding claims **characterized in that** the one or more non-ionic polymer(s) of composition A as compound(s) according to group b) are selected from one or more non-ionic carbohydrate-based hair styling polymer(s), preferably the one or more non-ionic polymer(s) are selected from one or more hydrolyzed starch polymer(s), more preferably the one or more non-ionic polymer(s) are selected from one or more hydrolyzed corn polymer(s), still more preferably the one or more non-ionic polymer(s) are selected from one or more hydrolyzed corn starch polymer(s).

5. The method according to any of the preceding claims **characterized in that** the total concentration of one or more compound(s) according to group b) in composition A is in the range of 0.5% to 15% by weight, preferably in the range of 0.75% to 10% by weight, more preferably in the range of 1% to 5% by weight, calculated to the total weight of composition A.

6. The method according to any of the preceding claims **characterized in that** the pH of composition A is in the range of 3 to 9, preferably the pH is in the range of 4 to 7, more preferably the pH is in the range of 4.5 to 6.5.

7. The method according to any of the preceding claims **characterized in that** composition A comprises water at 50% by weight, preferably at 75% by weight, more preferably at 80% by weight, calculated to the total weight of composition A.

8. The method according to any of the preceding claims **characterized in that** composition A comprises one or more foaming surfactant(s).

9. The method according to any of the preceding claims **characterized in that** composition A comprises one or more surfactant(s) selected from one or more non-ionic, anionic, cationic, or amphoteric/zwitterionic surfactant(s), and/or their mixtures, more preferably composition A comprises one or more non-ionic surfactant(s).

10. The method according to any of the preceding claims **characterized in that** the total concentration of organopolysiloxanes in composition A is 0.5% by weight or less, preferably 0.1% by weight or less, more preferably composition A is free of organopolysiloxanes.

11. The method according to any of the preceding claims **characterized in that** the keratin fibers are shampooed prior to application of composition A.

12. The method according to any of the preceding claims **characterized in that** composition A is applied onto wet or damp keratin fibers.

13. A kit-of-parts for caring and/or styling keratin fibers, preferably human keratin fibers, more preferably human hair, comprising:

- a composition B comprising one or more organic solvent(s) as compound(s) according to group a) as defined in any of the claims 1 to 12,
- a composition C comprising one or more non-ionic hair styling polymer(s) as compound(s) according to group b) as defined in any of the claims 1 to 12,

with the provision that at least one of the compositions B and/or C are aqueous compositions, and wherein the total concentration of anionic polymers in compositions B and C is 0.2% by weight or less, preferably 0.1% by weight or less, more preferably 0.05% by weight or less, calculated to the total weight of each of the compositions B and C, more preferably compositions B and C are free of anionic polymers.

14. A dispenser comprising the composition A as defined in any of the claims 1 to 12 or the compositions B and C as defined in claim 13.

15. The dispenser according to claim 14 **characterized in that** the dispenser is an aerosol vaporizer or a non-aerosol vaporizer such as a pump spray dispenser, a pump foam dispenser or a squeeze foam dispenser, preferably the dispenser is a non-aerosol vaporizer such as a pump foam dispenser, more preferably the dispenser is a pump spray or pump foam dispenser.


**Amended claims in accordance with Rule 137(2) EPC.**

1. A method for caring and/or styling keratin fibers, preferably human keratin fibers, more preferably human hair, comprising:

    i) applying to keratin fibers an aqueous composition A comprising:

        a) one or more organic solvent(s),
        b) one or more non-ionic hair styling polymer(s),

    ii) leaving the composition onto the keratin fibers and drying the keratin fibers, and
    iii) mechanically treating the keratin fibers, preferably mechanically curling the keratin fibers, more preferably mechanically curling the keratin fibers by scrunching, squeezing, or twisting the keratin fibers,
    wherein the total concentration of anionic polymers in composition A is 0.2% by weight or less, preferably 0.1% by weight or less, more preferably 0.05% by weight or less, calculated to the total weight of the composition A, more preferably composition A is free of anionic polymers,
    **characterized in that** the one or more non-ionic polymer(s) of composition A as compound(s) according to group b) are selected from one or more non-ionic carbohydrate-based hair styling polymer(s).

2. The method according to claim 1 **characterized in that** the one or more organic solvent(s) as compound(s) according to group a) is/are selected from monohydric, dihydric, and/or trihydric alcohol(s), and/or their mixtures, preferably the one or more organic solvent(s) as compound(s) according to group a) is/are selected from ethanol, propanol, isopropanol, butanol, isobutanol, ethoxydiglycol, 1,2-propanediol, 1,3-propanediol, and glycerol, and/or their mixtures, more preferably the one or more organic solvent(s) as compound(s) according to group a) is/are selected from 1,3-propanediol, ethoxydiglycol, and glycerol, and/or their mixtures, still more preferably the one or more organic solvent(s) of composition A as compound(s) according to group a) is/are 1,3-propanediol, glycerol, and/or their mixtures.

3. The method according to any of the claims 1 and/or 2 **characterized in that** the total concentration of the one or more compound(s) according to group a) in composition A, preferably the total concentration of 1,3-propanediol in composition A, is in the range of 0.1% to 10% by weight, preferably in the range of 0.25% to 7.5% by weight, more preferably in the range of 0.5% to 5% by weight, calculated to the total weight of the composition A.

4. The method according to any of the preceding claims **characterized in that** the one or more non-ionic polymer(s) of composition A as compound(s) according to group b) are selected from one or more hydrolyzed starch polymer(s), more preferably the one or more non-ionic polymer(s) are selected from one or more hydrolyzed corn polymer(s), still more preferably the one or more non-ionic polymer(s) are selected from one or more hydrolyzed corn starch polymer(s).

5. The method according to any of the preceding claims **characterized in that** the total concentration of one or more compound(s) according to group b) in composition A is in the range of 0.5% to 15% by weight, preferably in the range of 0.75% to 10% by weight, more preferably in the range of 1% to 5% by weight, calculated to the total weight of composition A.

6. The method according to any of the preceding claims **characterized in that** the pH of composition A is in the range of 3 to 9, preferably the pH is in the range of 4 to 7, more preferably the pH is in the range of 4.5 to 6.5.

7. The method according to any of the preceding claims **characterized in that** composition A comprises water at 50% by weight, preferably at 75% by weight, more preferably at 80% by weight, calculated to the total weight of composition A.

8. The method according to any of the preceding claims **characterized in that** composition A comprises one or more foaming surfactant(s).

9. The method according to any of the preceding claims **characterized in that** composition A comprises one or more surfactant(s) selected from one or more non-ionic, anionic, cationic, or amphoteric/zwitterionic surfactant(s), and/or their mixtures, more preferably composition A comprises one or more non-ionic surfactant(s).

10. The method according to any of the preceding claims **characterized in that** the total concentration of organopolysiloxanes in composition A is 0.5% by weight or less, preferably 0.1% by weight or less, more preferably composition A is free of organopolysiloxanes.

11. The method according to any of the preceding claims **characterized in that** the keratin fibers are shampooed prior to application of composition A.

12. The method according to any of the preceding claims **characterized in that** composition A is applied onto wet or damp keratin fibers.

13. A kit-of-parts for caring and/or styling keratin fibers, preferably human keratin fibers, more preferably human hair, comprising:

- a composition B comprising one or more organic solvent(s) as compound(s) according to group a) as defined in any of the claims 1 to 12,
- a composition C comprising one or more non-ionic hair styling polymer(s) as compound(s) according to group b) as defined in any of the claims 1 to 12,
with the provision that at least one of the compositions B and/or C are aqueous compositions, and
wherein the total concentration of anionic polymers in compositions B and C is 0.2% by weight or less, preferably 0.1% by weight or less, more preferably 0.05% by weight or less, calculated to the total weight of each of the compositions B and C, more preferably compositions B and C are free of anionic polymers.

14. A dispenser comprising the composition A as defined in any of the claims 1 to 12 or the compositions B and C as defined in claim 13.

15. The dispenser according to claim 14 **characterized in that** the dispenser is an aerosol vaporizer or a non-aerosol vaporizer such as a pump spray dispenser, a pump foam dispenser or a squeeze foam dispenser, preferably the dispenser is a non-aerosol vaporizer such as a pump foam dispenser, more preferably the dispenser is a pump spray or pump foam dispenser.

Europäisches
Patentamt

European
Patent Office

Office européen
des brevets

# EUROPEAN SEARCH REPORT

Application Number

EP 23 17 0654

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 792 600 A1 (KPSS KAO GMBH [DE]) 6 June 2007 (2007-06-06) | 1-3,5,6, 8-12,14, 15 | INV. A61Q5/06 A61K8/34 A61K8/73 |
| Y | * paragraphs [0009] - [0011], [0063] - [0065] * <br> * examples 1, 4, 5 * <br> * claims 1, 13, 15, 16 * | 7 | |
| X | WO 2022/251527 A1 (OREAL [FR]; FEREBEE MAHER RACHEL [US]) 1 December 2022 (2022-12-01) | 1-3,5,6, 10-12,14 | |
| Y | * paragraph [0113]; example 2H; table 2 * <br> * example 5C; table 5 * <br> * claim 24 * | 7 | |
| X | US 8 784 785 B2 (HENTRICH DIRK [DE]; EMMERLING WINFRIED [DE] ET AL.) 22 July 2014 (2014-07-22) | 1,2,4-6, 9-12,14, 15 | |
| Y | * claims 1-5 * <br> * examples 1, 2 * | 7 | |
| X | EP 1 800 715 A1 (HENKEL KGAA [DE]) 27 June 2007 (2007-06-27) | 1,2,5, 11-14 | TECHNICAL FIELDS SEARCHED (IPC) A61Q A61K |
| Y | * paragraph [0511] * <br> * claims 1, 4 * | 7 | |

The present search report has been drawn up for all claims

| Place of search | Date of completion of the search | Examiner |
|---|---|---|
| Munich | 15 September 2023 | Diebold, Alain |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 17 0654

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2023

| Patent document cited in search report | | Publication date | Patent family member(s) | | Publication date |
|---|---|---|---|---|---|
| EP 1792600 | A1 | 06-06-2007 | AT 421869 T | | 15-02-2009 |
| | | | EP 1792600 A1 | | 06-06-2007 |
| WO 2022251527 | A1 | 01-12-2022 | NONE | | |
| US 8784785 | B2 | 22-07-2014 | AU 2005244611 A1 | | 01-12-2005 |
| | | | US 2008230084 A1 | | 25-09-2008 |
| | | | WO 2005112878 A1 | | 01-12-2005 |
| | | | WO 2005115319 A1 | | 08-12-2005 |
| EP 1800715 | A1 | 27-06-2007 | DE 102005061917 A1 | | 05-07-2007 |
| | | | EP 1800715 A1 | | 27-06-2007 |

EPO FORM P0459

For more details about this annex : see Official Journal of the European Patent Office, No. 12/82